# FASCICULE DE BREVET EUROPEEN

(11) **EP 3 454 959 B1**
(45) Date de publication et mention de la délivrance du brevet: **12.08.2020**
(21) Numéro de dépôt: 17722033.2
(22) Date de dépôt: 09.05.2017
(51) Int. Cl.: B01D 11/04, C07C 7/10, C07C 1/20, C07C 29/86

(54) **UTILISATION D'UN SOLVANT DONNEUR DE LEWIS POUR LA PURIFICATION D'UNE CHARGE COMPRENANT DE L'ETHANOL, DE L'ACETALDEHYDE, ET DES IMPURETES**
VERWENDUNG EINES LEWIS-DONATOR-LÖSUNGSMITTELS ZUR REINIGUNG EINES ROHSTOFFS MIT ETHANOL, ACETALDEHYD UND VERUNREINIGUNGEN
USE OF A LEWIS DONOR SOLVENT FOR PURIFYING A FEEDSTOCK COMPRISING ETHANOL, ACETALDEHYDE, AND IMPURITIES

(30) Priorité: 12.05.2016 FR 1654234
(43) Date de publication de la demande: 20.03.2019
(73) Titulaire: IFP Energies nouvelles, 92852 Rueil-Malmaison (FR); Compagnie Générale des Etablissements Michelin, 63000 Clermont-Ferrand (FR)
(72) Inventeur: HOGNON, Céline, 69780 Mions (FR); DROZDZ, Sophie, 69126 Brindas (FR); JACQUIN, Marc, 69002 Lyon (FR)
(86) Numéro de dépôt international: PCT/EP2017/061094
(87) Numéro de publication internationale: WO 2017/194559

(56) Documents cités:
- FR-A1- 3 014 098
- FR-A1- 3 026 100
- FR-A1- 3 026 101
- US-A- 2 403 743

## Description

### DOMAINE TECHNIQUE DE L'INVENTION

La présente invention concerne un nouveau solvant d'extraction liquide-liquide pour l'élimination des impuretés dans un mélange aqueux d'éthanol et d'acétaldéhyde ainsi que le procédé mettant en œuvre ce solvant.

### ART ANTERIEUR

Les procédés de production de butadiène à partir d'éthanol ont été développés, en particulier, par des équipes américaines durant la seconde guerre mondiale à partir des travaux d'Ostromilenski.

Dans ce procédé, la conversion par passe est inférieure à 50%, ce qui implique des recyclages importants de l'éthanol et de l'acétaldéhyde. De plus, par ce procédé, une grande variété d'impuretés de différentes natures (hydrocarbures saturés, insaturés, aromatiques, produits oxygénés [alcools, cétones, aldéhydes, phénols, acides, esters, éthers]) et ayant des masses molaires très différentes est produite (entre 50 et 10 000 g/mol).

Il est donc nécessaire de mettre en place un enchaînement d'opérations unitaires dans le but d'éliminer le maximum d'impuretés en perdant le moins possible d'éthanol et d'acétaldéhyde. D'un point de vue économique, il est primordial de diminuer le coût de production du butadiène, ce qui nécessite de :
- perdre le moins possible d'éthanol et d'acétaldéhyde
- ne pas recycler d'impuretés dans les réacteurs qui induiraient une chute de sélectivité en butadiène, ou s'accumuleraient à des niveaux non acceptables, nécessitant une purge et donc des pertes en éthanol et acétaldéhyde.

À la sortie des réacteurs catalytiques, l'effluent produit composé de butadiène, d'acétaldéhyde, d'eau, d'éthanol et d'impuretés subit plusieurs opérations unitaires afin de séparer les sous-produits gazeux à température ambiante des sous-produits liquides à température ambiante.

Parmi les sous-produits gazeux, on peut citer l'hydrogène, le monoxyde de carbone, le dioxyde de carbone, les alcanes et les oléfines en C₁-C₄. Il est primordial d'éliminer ces sous-produits de l'effluent riche en butadiène afin d'obtenir un produit aux spécifications.

Parmi les sous-produits liquides à température ambiante, on peut citer l'acétone, le diéthyléther, le butanal, le butanol, le butanone, l'éthyle acétate, le crotonaldéhyde et l'acide acétique. D'autres sous-produits sont produits en plus faible quantité dans la zone réactionnelle. Dans la suite du document, on désignera par « impuretés » cet ensemble de milliers de composés hydrocarbonés ou oxygénés.

Dans les premiers schémas de procédé des équipes américaines, l'éthanol, l'acétaldéhyde, l'eau et les sous-produits liquides étaient séparés par un enchaînement de trois colonnes à distiller (brevet US 2,403,742). L'effluent riche en éthanol, acétaldéhyde, eau et sous-produits liquides alimente une première colonne à distiller dans laquelle un effluent riche en acétaldéhyde est séparé du reste de l'effluent. Une seconde colonne à distiller permet de séparer les sous-produits liquides d'un effluent riche en éthanol et eau. La dernière colonne à distiller permet de séparer l'éthanol de l'eau. La majeure partie des brevets de procédé déposés dans la période 1940-1960 par les sociétés Carbide & Carbon ou Koppers (US 2,403,743 ; US 2,393,381 ; US 2,395,057 et US 2,439,587) visent à améliorer cette partie du schéma.

Les impuretés liquides formant un continuum de volatilité de très léger à très lourd, il est très difficile de les isoler parfaitement de l'éthanol et de l'acétaldéhyde. De plus, la nature des impuretés allant de très hydrophobe à très hydrophile, des séparations de phase se produisent aussi bien dans le résidu que dans le distillat ce qui entraine des complications de mise en œuvre au sein des colonnes à distiller.

Dans les brevets FR 1,458,859, FR 3 026 100, FR 3 026 101 et FR 1,458,860, l'élimination des impuretés liquides se fait par extraction liquide-liquide. L'effluent composé d'éthanol, d'acétaldéhyde, d'eau et d'impuretés alimente une colonne d'extraction liquide-liquide. Cette dernière est alimentée en fond par un solvant de lavage qui a pour but de laver à contre-courant la charge. A la sortie de cette section de lavage, l'extrait est composé majoritairement du solvant de lavage, des sous-produits extraits et d'une faible quantité d'éthanol et d'acétaldéhyde. Cet extrait est ensuite lavé à l'eau dans le but de ré-extraire l'éthanol et l'acétaldéhyde et ainsi minimiser les pertes en éthanol et acétaldéhyde. Le solvant de lavage employé pour cette opération unitaire est constitué d'un mélange d'hydrocarbures ayant entre 6 et 40 atomes de carbones, de préférence entre 10 et 20 atomes de carbone. Le solvant de lavage peut être une coupe gazole ou kérosène désulfurée ou bien encore une coupe d'hydrocarbures produite par une unité de type Fischer-Tropsch.

La régénération du solvant de lavage est réalisée par deux colonnes de distillation en série. La première, intitulée dans le brevet FR 1,458,859 « section de distillation des huiles brunes légères », sépare les impuretés légères d'un résidu hydrocarboné comprenant les impuretés lourdes et le solvant de lavage. Ce résidu est ensuite distillé dans une seconde colonne intitulée « section de distillation des huiles lourdes » produisant comme distillat un mélange composé essentiellement du solvant de lavage avec quelques traces d'impuretés et en tant que résidu l'effluent intitulé « huiles brunes lourdes » comprenant les impuretés lourdes.

Dans cette configuration, lors de l'extraction liquide-liquide un précipité se forme et peut à terme encrasser les colonnes d'extraction, les pales d'agitation ou le garnissage. Pour pallier ce problème il est nécessaire d'utiliser un matériau anti-encrassement assez onéreux ou arrêter le système régulièrement pour tout nettoyer.

### OBJET ET INTERET DE L'INVENTION

L'invention concerne l'utilisation d'un solvant spécifique pour l'élimination des impuretés contenues dans une charge comprenant de l'eau, de l'éthanol, de l'acétaldéhyde et des impuretés, ainsi qu'un procédé mettant en œuvre ce solvant.

De manière surprenante, les demanderesses ont découvert que l'utilisation de solvant ayant des propriétés de donneur de Lewis permettait d'obtenir des performances améliorées, en particulier par rapport aux solvants décrits dans les brevets FR 1,458,859 , FR 3 026 100, FR 3 026 101 et FR 1,458,860. En effet, la mise en œuvre des solvants identifiés par les demanderesses permet d'éviter la formation d'un précipité qui est néfaste au bon fonctionnement de la colonne d'extraction liquide-liquide. Par ailleurs, ces solvants permettent de réduire le ratio entre le débit de solvant et le débit de charge tout en maintenant une bonne qualité de séparation entre les impuretés d'une part, et l'éthanol et l'acétaldéhyde d'autre part.

### DESCRIPTION DETAILLEE DE L'INVENTION

L'invention concerne un procédé de purification d'une charge comprenant de l'éthanol, de l'acétaldéhyde et des impuretés par un solvant, appelé solvant donneur de Lewis, comprenant un composé donneur de Lewis choisi dans le groupe constitué par les acides gras insaturés ayant entre 12 et 18 atomes de carbone, les esters de phosphate ayant entre 12 et 30 atomes de carbone, et leurs mélanges comprenant :
- une étape A) d'extraction liquide-liquide à contre-courant alimentée en tête par ladite charge en mélange avec le raffinat issu de l'étape B) de réextraction, ce mélange constituant l'alimentation de ladite étape A), et en fond par l'effluent riche en solvant donneur de Lewis issu de l'étape C) de régénération, un appoint en solvant donneur de Lewis étant éventuellement mélangé audit effluent riche en solvant donneur de Lewis, et produisant en tête un extrait et en fond une charge purifiée, opérée à une température comprise entre 10 et 70°C et à une pression comprise entre 0,1 et 0,5 MPa avec un rapport débit massique de phase continue / débit massique de phase dispersée inférieur à 70 ;
- une étape B) de réextraction liquide-liquide à contre-courant alimentée en tête par un solvant auxiliaire et en fond par l'extrait issu de l'étape A), et produisant en tête un extrait et en fond un raffinat, ledit raffinat alimentant ladite étape A), opérée à une température comprise entre 10 et 70°C et à une pression comprise entre 0,1 et 0,5 MPa avec un rapport de débit massique de phase continue / débit massique de phase dispersée inférieur à 70;
- une étape C) de régénération dans laquelle l'extrait issu de l'étape B) est séparé par une première distillation en un distillat riche en impuretés légères et un résidu qui subit une seconde distillation, laquelle produit en tête un effluent riche en solvant donneur de Lewis et un résidu riche en impuretés lourdes.

L'invention concerne également l'utilisation d'un solvant comprenant un composé donneur de Lewis choisi dans le groupe constitué par les acides gras insaturés ayant entre 12 et 18 atomes de carbone, les esters de phosphate ayant entre 12 et 30 atomes de carbone, et leurs mélanges pour la séparation des impuretés d'une charge comprenant de l'éthanol, de l'acétaldéhyde, et des impuretés.

L'invention concerne également l'utilisation d'un solvant comprenant un composé donneur de Lewis choisi dans le groupe constitué par les acides gras insaturés ayant entre 12 et 18 atomes de carbone, les esters de phosphate ayant entre 12 et 30 atomes de carbone, et leurs mélanges pour la séparation des impuretés d'une charge comprenant de l'éthanol, de l'acétaldéhyde, et des impuretés selon les étapes dudit procédé de purification d'une charge comprenant de l'éthanol, de l'acétaldéhyde et des impuretés.

Ledit solvant comprenant un composé donneur de Lewis utilisé selon l'invention ou mis en œuvre dans le procédé selon l'invention permet d'éliminer les impuretés contenues dans une charge comprenant de l'éthanol, de l'acétaldéhyde et des impuretés. Les impuretés sont de natures très variées (hydrocarbures saturés, insaturés, aromatiques, produits oxygénés, parmi lesquels alcools, cétones, aldéhydes, composés phénoliques, acides, esters, éthers) et ayant des masses molaires allant de 50 à plus de 10 000 g/mol. Parmi les impuretés typiques, on peut citer l'acétone, le diéthyléther, le butanal, des butanols, des butanones, l'éthyle acétate, le crotonadélhyde, des pentènes, des pentadiènes, des hexènes et des hexadiènes.

De manière connue de l'homme du métier, on entend par « composé donneur de Lewis », ou « base de Lewis », un composé présentant un doublet d'électrons qui peut être déplacé vers un autre composé, appelé, lui, « acide de Lewis ». Pour les besoins de l'invention, le composé donneur de Lewis est choisi dans le groupe constitué par les acides gras insaturés ayant entre 12 et 18 atomes de carbone, les esters de phosphate ayant entre 12 et 30 atomes de carbone, et leurs mélanges. Ledit composé donneur de Lewis est préférentiellement choisi dans le groupe constitué par l'acide oléique, l'acide linoléique, le tributylphosphate et leurs mélanges. Plus préférentiellement, le composé donneur de Lewis est l'acide oléique.

Selon un mode préféré, le solvant comprenant un composé donneur de Lewis est constitué de 100% en poids d'un composé donneur de Lewis choisi dans le groupe constitué par les acides gras insaturés ayant entre 12 et 18 atomes de carbone, les esters de phosphate ayant entre 12 et 30 atomes de carbone, et leurs mélanges.

Selon un autre mode, également préféré, ledit solvant donneur de Lewis comprend également au moins un hydrocarbure contenant au moins 12 atomes de carbone. La proportion (composé donneur de Lewis)/(hydrocarbure + composé donneur de Lewis) est comprise dans un domaine allant de 1% à moins de 100% poids, de préférence de 15% à moins de 100% poids et de manière très préféré de 30% à moins de 100% poids.

Ledit hydrocarbure est sélectionné de manière à n'être pas soluble dans la charge alimentant ladite étape A). Ledit hydrocarbure est avantageusement une coupe C₁₂-C₅₀, très avantageusement une coupe C₁₄-C₃₀. Ledit hydrocarbure est avantageusement une coupe gasoil ou un fioul. Avantageusement, la coupe hydrocarbure est sélectionnée de manière à ce que la température du solvant donneur de Lewis soit comprise dans la gamme de température d'ébullition de la coupe hydrocarbure.

### Charge à purifier

La charge à purifier par le procédé selon l'invention, ou à purifier par l'utilisation selon l'invention est une solution aqueuse comprenant de l'éthanol, de l'acétaldéhyde, et des impuretés, ces dernières devant être éliminées.

De préférence, la teneur en éthanol dans ladite charge est comprise entre 40 % et 70% poids, de préférence entre 50 et 60% poids, la teneur en acétaldéhyde est comprise entre 1 et 30% poids, de préférence entre 5 et 10% poids et la teneur en impuretés est comprise entre 1 et 20% poids, de préférence entre 5 et 20% poids.

### Étape A) d'extraction des impuretés

Le procédé de purification selon l'invention comprend une étape A) d'extraction liquide-liquide à contre-courant alimentée en tête par ladite charge en mélange avec le raffinat issu de l'étape B) de réextraction, ce mélange constituant l'alimentation de ladite étape A), et en fond par l'effluent riche en solvant donneur de Lewis spécifique tel que décrit ci-dessus, issu de l'étape C) de régénération, et produisant en tête un extrait et en fond une charge purifiée, opérée à une température comprise entre 10 et 70°C et à une pression comprise entre 0,1 et 0,5 MPa, préférentiellement entre 0,2 et 0,4 MPa avec un rapport de débit massique de phase continue / débit massique de phase dispersée inférieur à 70, de préférence inférieur à 3, de préférence inférieur à 1,5. Au-delà de 70, le fonctionnement hydrodynamique de la colonne est compromis. Peu importe que le solvant donneur de Lewis forme la phase continue ou dispersée, ce critère étant un critère hydrodynamique.

Comme connu de l'Homme du métier, une extraction liquide-liquide fonctionne avec deux phases liquides, l'une des phases constituant la phase continue et l'autre constituant la phase dispersée, présente sous forme de gouttes distinctes. La nature continue ou dispersée dépend du débit relatif d'une phase par rapport à l'autre. Ainsi, selon le phénomène bien connu, si l'on réduit le débit de la phase continue en augmentant le débit de la phase dispersée, la phase dispersée deviendra continue et inversement.

Le solvant donneur de Lewis tourne en rond entre les étapes A), B) et C). Les pertes éventuelles en solvant donneur de Lewis sont compensées par un apport extérieur en solvant donneur de Lewis mélangé à l'effluent riche en solvant donneur de Lewis issu de l'étape C). Lors du démarrage du procédé selon l'invention, le solvant donneur de Lewis est alimenté en fond de ladite étape A), le débit d'alimentation étant réduit au fur et à mesure que le recyclage en effluent riche en solvant donneur de Lewis issu de l'étape C) se met en place jusqu'à ne plus représenter que le débit d'appoint éventuel.

L'effluent riche en solvant donneur de Lewis issu de l'étape C) et la charge alimentant ladite étape A) sont alimentés chacun à une température indépendamment comprise entre 10 et 70°C, préférentiellement entre 40 et 55°C.

Plus le rapport débit massique d'effluent riche en solvant issu de l'étape C) / débit massique de l'alimentation de ladite étape A) est élevé, plus l'étape d'extraction des impuretés est efficace. Cependant, un ratio élevé conduit à extraire également une fraction importante d'éthanol et d'acétaldéhyde dans l'extrait de ladite étape A), et par conséquent à augmenter le débit de solvant auxiliaire nécessaire au sein de l'étape B) pour limiter les pertes en éthanol et acétaldéhyde. La valeur du ratio de débit massique d'effluent riche en solvant donneur de Lewis sur le débit massique de solvant auxiliaire doit donc être ajusté de manière à extraire le maximum d'impuretés tout en limitant les pertes en éthanol et acétaldéhyde. Plus ce ratio est élevé, plus on extraira d'impuretés dans l'extrait issu de l'étape B) et plus les pertes en éthanol et acétaldéhyde, c'est-à-dire l'éthanol et l'acétaldéhyde que l'on trouve dans ledit extrait issu de l'étape B), seront importantes.

Le ratio de débit massique d'effluent riche en solvant donneur de Lewis sur le débit massique de solvant auxiliaire est ajusté de manière à ce que ledit extrait issu de l'étape B) comprenne 50% poids, de préférence 60% poids et de manière préférée 70% poids des impuretés contenues dans ladite charge, ainsi qu'au plus 5% poids, de préférence au plus 2% poids, et de manière préférée au plus 1% poids de la quantité totale d'éthanol et d'acétaldéhyde contenue dans ladite charge.

Le contact entre les deux phases liquides dans ladite section d'extraction est réalisé au sein d'un extracteur liquide-liquide. Différents modes de contact peuvent être envisagés. On peut citer de manière non limitative, une colonne garnie, une colonne puisée, une colonne compartimentée agitée ou bien une batterie de mélangeur-décanteur.

Ledit extrait de ladite étape A) alimente l'étape B) de ré-extraction de l'éthanol et de l'acétaldéhyde.

De manière surprenante, la demanderesse a découvert que l'utilisation d'un solvant d'extraction spécifique tel que décrit ci-dessus, non miscible avec une charge comprenant de l'éthanol et de l'acétaldéhyde, permettait d'éviter largement l'encrassement de la colonne.

L'utilisation du solvant d'extraction selon l'invention permet de réduire les coûts opératoires en réduisant la quantité de solvant et en évitant l'encrassement dans les étapes A) et B).

### Etape B) de ré-extraction de l'éthanol et de l'acétaldéhyde

Le procédé de purification selon l'invention comprend une étape B) de réextraction liquide-liquide à contre-courant, avantageusement réalisée au sein d'un extracteur liquide-liquide, et alimentée :
- en tête par un solvant auxiliaire,
- en fond par l'extrait issu de l'étape A),
opérant à une température comprise entre 10 et 70°C et à une pression comprise entre 0,1 et 0,5 MPa, préférentiellement entre 0,2 et 0,4 MPa, avec un rapport de débit massique de phase continue / débit massique de phase dispersée inférieur à 70, de préférence inférieur à 3, de préférence inférieur à 1,5, au-delà de 70, le fonctionnement hydrodynamique de la colonne étant compromis, et produisant :
- en tête un extrait comprenant avantageusement moins de 5% de la quantité totale d'éthanol et d'acétaldéhyde contenue dans ladite charge,
- en fond un raffinat.

Peu importe que le solvant auxiliaire forme la phase continue ou dispersée, ce critère étant un critère hydrodynamique.

Ledit solvant auxiliaire est une solution aqueuse comprenant au moins 90% poids d'eau, de préférence de l'eau d'origine externe au procédé et, de préférence, exempt d'éthanol et d'acétaldéhyde. L'utilisation d'eau permet de diminuer la teneur en éthanol et acétaldéhyde dans l'extrait de la section B) et d'améliorer la séparation entre les impuretés et les composés éthanol et acétaldéhyde dans la section d'extraction A) en abaissant le ratio éthanol + acétaldéhyde / eau dans ladite section. Ledit solvant auxiliaire peut, par exemple, être un flux d'eau issu d'un procédé de conversion de l'éthanol en butadiène.

Ledit solvant auxiliaire et ledit extrait issu de l'étape A) sont alimentés indépendamment à une température comprise entre 10 et 70°C, préférentiellement entre 40 et 55°C.

Le raffinat produit en fond de ladite étape B) est mélangé à la charge alimentant le procédé selon l'invention et peut alimenter ainsi ladite étape A) en tête.

Le contact entre les deux phases liquides dans ladite étape de réextraction est avantageusement réalisé au sein d'un extracteur liquide-liquide. Différents modes de contact peuvent être envisagés. On peut citer de manière non limitative, une colonne à garnissage, une colonne puisée, une colonne compartimentée agitée ou bien une batterie de mélangeur-décanteur.

Les étapes A) et B) sont avantageusement mises en œuvre dans un seul équipement.

Ledit extrait issu de l'étape B) de ré-extraction alimente la section de régénération C).

### Étape C) de régénération du solvant

Le procédé de purification selon l'invention comprend une étape C) de régénération dans laquelle l'extrait issu de l'étape B) de ré-extraction liquide-liquide alimente une première colonne de distillation qui produit en tête un distillat riche en impuretés qui est éliminé du procédé et en fond un résidu qui alimente une seconde colonne à distiller. Ladite seconde colonne à distiller produit en fond un résidu riche en impuretés qui est éliminé du procédé et en tête un effluent riche en solvant donneur de Lewis, ledit effluent étant ensuite recyclé dans l'étape A) d'extraction liquide-liquide.

Les impuretés peuvent être classées :
1) en «impuretés légères», dont la température d'ébullition est inférieure à la température d'ébullition du solvant donneur de Lewis et qui sont produites en tête de la première colonne à distiller ;
2) et en « impuretés lourdes », dont la température d'ébullition est supérieure à la température d'ébullition du solvant donneur de Lewis, et qui sont produites en fond de ladite seconde colonne à distiller.
Ces impuretés (légères et lourdes) correspondent à un ensemble de milliers de composés hydrocarbonés ou oxygénés.

Ledit distillat de la première colonne à distiller est majoritairement composé d'« impuretés légères ». Par majoritairement, on entend plus de 85% pds, de préférence plus de 95% pds. Ledit distillat riche en impuretés peut être brulé pour fournir une partie de la chaleur nécessaire au circuit d'huile chaud ou aux chaudières à vapeur du procédé de production de butadiène à partir d'éthanol et d'acétaldéhyde.

Ledit effluent de la seconde colonne à distiller est majoritairement composé du solvant donneur de Lewis. Par majoritairement, on entend plus de 85% poids, avantageusement plus de 95% poids. Ledit effluent riche en solvant donneur de Lewis est recyclé en fond de ladite étape A) d'extraction liquide-liquide.

Ledit résidu de la seconde colonne à distiller est majoritairement composé d'« impuretés lourdes». Par majoritairement, on entend plus de 85% pds, de préférence plus de 95% pds. Ledit résidu riche en impuretés peut être brûlé pour fournir une partie de la chaleur nécessaire au circuit d'huile chaud ou aux chaudières à vapeur du procédé de production de butadiène à partir d'éthanol et d'acétaldéhyde.

Dans un autre mode de l'invention, adapté au cas de figure où la proportion d'impuretés lourdes est faible, seule une fraction du résidu de ladite première colonne de distillation alimente de manière continue ladite seconde colonne de distillation, la fraction restante étant recyclée vers ladite étape A) en tant qu'effluent riche en solvant donneur de Lewis.

Dans un autre mode de l'invention, adapté au cas de figure où la proportion d'impuretés lourdes est très faible, seule une fraction du résidu de ladite première colonne de distillation alimente de manière séquentielle ladite seconde colonne de distillation, la fraction restante étant recyclée vers ladite étape A) en tant qu'effluent riche en solvant donneur de Lewis.

L'utilisation d'un solvant ayant une température d'ébullition élevée permet de réduire les coûts opératoires et les coûts d'investissement de la section C) de régénération du solvant par rapport à l'art antérieur en réduisant la taille ou la fréquence d'utilisation de ladite seconde colonne de distillation.

### DESCRIPTION DES FIGURES

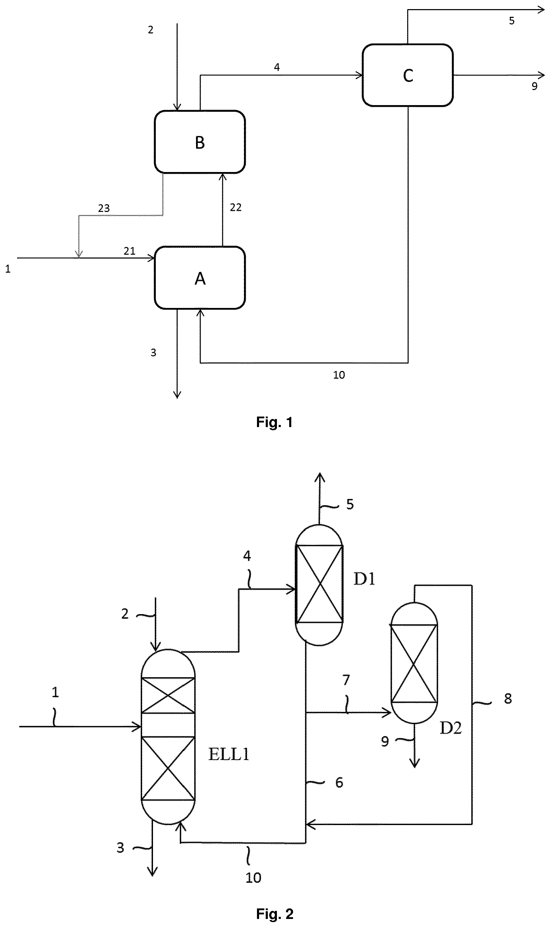

La **fig.1** représente de manière schématique un arrangement général du procédé selon l'invention.
   Une étape A) d'extraction liquide-liquide à contre-courant est alimentée par une charge (1) en mélange avec le raffinat (23) issu de l'étape B) de réextraction, ce mélange constituant l'alimentation (21) de ladite étape A), et en fond par l'effluent riche en solvant donneur de Lewis (10) issu de l'étape C) de régénération, et produisant un extrait (22) et une charge purifiée (3).
   Une étape B) de réextraction liquide-liquide à contre-courant est alimentée par un solvant auxiliaire (2) et en fond par l'extrait (22) issu de l'étape A), et produit en tête un extrait (4) et en fond un raffinat, (23) ledit raffinat alimentant ladite étape A).
   Une étape C) de régénération est alimentée par l'extrait (4) issu de l'étape B) et sépare un distillat riche en impuretés légères (5), un effluent riche en solvant donneur de Lewis (10) et un résidu riche en impuretés lourdes (9).
La **fig.2** représente de manière schématique et non limitative un arrangement du procédé selon l'invention. La numérotation des courants de la **fig.2** est identique à celle de la **fig.1****.**
   Dans l'arrangement présenté **fig.2****,** les étapes A) et B) sont mises en œuvre dans un seul équipement ELL1.
   La charge comprenant de l'éthanol et de l'acétaldéhyde (1) alimente une colonne d'extraction liquide-liquide ELL1. Cette dernière est alimentée en tête par un solvant auxiliaire (2) et en fond par l'effluent riche en solvant donneur de Lewis issu de l'étape C) (10). L'extrait (4) de l'étape B) est soutiré en tête de la colonne ELL1 tandis qu'en fond de la colonne ELL1, La charge purifiée (3) de l'étape A) est soutirée.
   Dans l'arrangement présenté **fig.2****,** l'étape C) est mise en œuvre dans deux colonnes à distiller D1 et D2.

L'extrait (4) issu de l'étape B) alimente une colonne à distiller D1. Un distillat riche en impuretés légères (5) est séparé en tête et un résidu est soutiré en fond. Lorsque la proportion d'impuretés lourdes dans ce résidu est faible, seule une fraction (7) dudit résidu alimente une seconde colonne à distiller D2, la fraction résiduelle (6) étant recyclée vers l'étape A) dans la colonne ELL1 en mélange avec le flux (8) en tant qu'effluent riche en solvant donneur de Lewis (10).

Un appoint éventuel (non représenté) en solvant donneur de Lewis peut être réalisé en mélange avec l'effluent riche en solvant donneur de Lewis (10) pour compenser les pertes en donneur de Lewis, ou lors du démarrage du procédé.

La seconde colonne à distiller D2 sépare en tête un effluent riche en solvant donneur de Lewis (8), recyclé vers l'étape A) dans la colonne ELL1 en mélange avec la fraction résiduelle (6), et en fond un résidu riche en impuretés lourdes (9) qui est éliminé du procédé.

### EXEMPLES

### Exemple 1

*Dans cet exemple, on compare les performances obtenues avec un solvant conforme à l'invention à celles obtenues avec les solvants décrits dans l'art antérieur pour un ratio massique solvant*/*charge donné.*

On analyse les performances obtenues pour purifier des impuretés clés d'une charge dont la composition est similaire à celle d'un effluent industriel d'un procédé de type Lebedev (éthanol = 50 % poids; acétaldéhyde = 9 % poids ; eau = 27 % poids, impuretés = 14 % poids). Le ratio massique solvant/charge est de 1.

On mesure le coefficient de partage K de l'acétate d'éthyle et du diéthyléther, entre une phase hydro-alcoolique et différents solvants testés. Par impuretés clés, on entend que la capacité à séparer ces impuretés est représentative de la capacité à séparer le mélange complexe d'impuretés dans un flux réel.

On note Kᵢ= [I]_{S1}/[I]_{S2}, où [I]_{S1} est la concentration de l'espèce l en mol/kg dans le solvant d'extraction, noté S1, et [l]_{S2} est une concentration de l'espèce l en mol/kg dans la phase hydro-alcoolique, noté S2.
On définit également la sélectivité d'absorption des impuretés i par rapport à l'éthanol S_{l/ethanol}=Kₗ/K_{éthanol}.

Différents solvants d'extraction non conformes ont été testés :
- Un hydrocarbure ayant moins de 12 atomes de carbone : l'hexane
- Un acide gras ayant moins de 12 atomes de carbone : l'acide nonanoïque
- Un ester de phosphate ayant moins de 12 atomes de carbone : le tri-éthyl-phosphate
- Un hydrocarbure ayant au moins 12 atomes de carbone : l'hexadécane

Ces solvants sont comparés au solvant conforme suivant :
- Un mélange d'un hydrocarbure et d'un composé donneur de Lewis ayant plus de 12 atomes de carbone : mélange acide oléique et hexadécane

Les coefficients de partage des deux impuretés clés (acétate d'éthyle et diéthyléther), leurs sélectivités par rapport à l'éthanol et la présence ou non d'un dépôt sont donnés dans le tableau ci-dessous et pour différents solvants, selon l'invention et selon l'art antérieur :

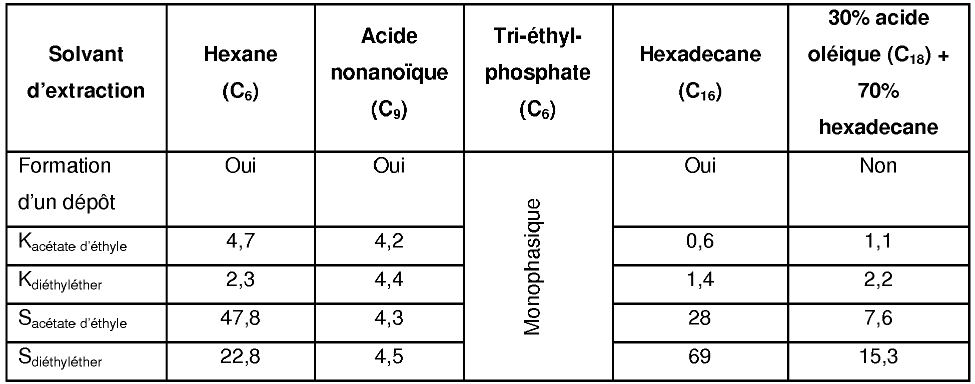

Dans le cas du tri-éthyl-phosphate, la séparation est impossible car le mélange est monophasique. L'hexane est un excellent solvant d'extraction, mais le mélange avec la charge n'est diphasique que dans des conditions étroites de concentrations, ce qui rend sa mise en œuvre en extraction liquide-liquide délicate.

On observe que l'utilisation conformément à l'invention d'un mélange hexadécane-acide oléique conduit à une meilleure extraction que l'hexadécane seul. De plus, bien que la sélectivité soit un peu plus faible, le mélange ne forme pas de dépôt dans l'équipement.

### Exemple 2

*Dans cet exemple, on compare les performances obtenues avec un solvant conforme à l'invention avec des solvants ayant des fonctions chimiques différentes pour un ratio massique solvant*/*charge donné*

On analyse les performances obtenues pour séparer les impuretés et les huiles brunes d'une charge proche d'une composition industrielle (éthanol = 50 % poids; acétaldéhyde = 9 % poids ; eau = 27 % poids, impuretés = 14 % poids). Le ratio massique solvant/charge est de 1.

Différents solvants d'extraction non-conformes ont été testés :
- Un ester méthylique d'acide gras composé de 18 atomes de carbone : l'ester méthylique de tournesol
- Un alcool composé de 18 atomes de carbone : l'alcool oléique
ainsi que les solvants conformes suivant :
- Un acide gras composé de 18 atomes de carbone et d'une double liaison : l'acide oléique
- Un acide gras composé de 18 atomes de carbone et de deux doubles liaisons : l'acide linoléique
- Un composé organophosphoré : le tributylphosphate, composé de 12 atomes de carbone.

Les coefficients de partage de deux impuretés clés (acétate d'éthyle, diéthyléther), leurs sélectivités par rapport à l'éthanol et la présence ou non d'un dépôt sont donnés dans le tableau ci-dessous et pour différents solvants, selon l'invention et selon l'art antérieur :

| **Solvant d'extraction** | **Ester méthylique de tournesol** | **Alcool oléique** | **Acide oléique** | **Acide linoléique** | **Tributylphosphate** |
|---|---|---|---|---|---|
| Formation d'un dépôt | Oui | Oui | Non | Non | Non |
| K_{acétate d'éthyle} | 1,8 | 2,0 | 2,1 | 2,5 | 1,7 |
| K_{diéthyléther} | 2,5 | 2,4 | 2,6 | 3,1 | 1,8 |
| S_{acétat**e** d'éthyle} | 4,0 | 3,6 | 2,8 | 3,6 | 1,8 |
| S_{diéthyléther} | 17,2 | 4,4 | 3,6 | 4,6 | 2,0 |

Cet exemple comparatif illustre bien l'intérêt d'utiliser un composé donneur de Lewis telle que prescrit pour les besoins de l'invention qui permet d'éviter la formation d'un dépôt.

## Revendications

1. Procédé de purification d'une charge comprenant de l'éthanol, de l'acétaldéhyde et des impuretés par un solvant, appelé solvant donneur de Lewis: Z
- une étape A) d'extraction liquide-liquide à contre-courant alimentée en tête par ladite charge en mélange avec le raffinat issu de l'étape B) de réextraction, ce mélange constituant l'alimentation de ladite étape A), et en fond par l'effluent riche en solvant donneur de Lewis issu de l'étape C) de régénération, un appoint en solvant donneur de Lewis étant éventuellement mélangé audit effluent riche en solvant donneur de Lewis, et produisant en tête un extrait et en fond une charge purifiée, opérée à une température comprise entre 10 et 70°C et à une pression comprise entre 0,1 et 0,5 MPa avec un rapport débit massique de phase continue / débit massique de phase dispersée inférieur à 70 ;
- une étape B) de réextraction liquide-liquide à contre-courant alimentée en tête par un solvant auxiliaire et en fond par l'extrait issu de l'étape A), et produisant en tête un extrait et en fond un raffinat, ledit raffinat alimentant ladite étape A), opérée à une température comprise entre 10 et 70°C et à une pression comprise entre 0,1 et 0,5 MPa avec un rapport de débit massique de phase continue / débit massique de phase dispersée inférieur à 70 ;
- une étape C) de régénération dans laquelle l'extrait issu de l'étape B) est séparé par une première distillation en un distillat riche en impuretés légères et un résidu qui subit une seconde distillation, laquelle produit en tête un effluent riche en solvant donneur de Lewis et un résidu riche en impuretés lourdes ; **caractérisé en ce que** Z
ledit solvant donneur de Lewis comprend un donneur de Lewis choisi dans le groupe constitué par les acides gras insaturés ayant entre 12 et 18 atomes de carbone, les esters de phosphate ayant entre 12 et 30 atomes de carbone et leurs mélanges.

2. Procédé selon la revendication précédente dans lequel ledit composé donneur de Lewis est choisi dans le groupe constitué par l'acide oléique, l'acide linoléique, le tributylphosphate et leurs mélanges.

3. Procédé selon la revendication 1 dans lequel ledit solvant donneur de Lewis comprend également au moins un hydrocarbure contenant au moins 12 atomes de carbone, la proportion (composé donneur de Lewis)/(hydrocarbure + composé donneur de Lewis) étant comprise dans un domaine allant de 1% à moins de 100% poids.

4. Procédé selon la revendication précédente dans lequel ledit ratio est compris dans un domaine allant de 15 à moins de 100% poids.

5. Procédé selon l'une des revendications 1 à 4 dans lequel ladite solution de solvant auxiliaire est de l'eau.

6. Utilisation d'un solvant comprenant un composé donneur de Lewis choisi dans le groupe constitué par les acides gras insaturés ayant entre 12 et 18 atomes de carbone, les esters de phosphate ayant entre 12 et 30 atomes de carbone, et leurs mélanges pour la séparation des impuretés d'une charge comprenant de l'éthanol, de l'acétaldéhyde, et des impuretés.

7. Utilisation selon la revendication 6 dans laquelle ledit composé donneur de Lewis est choisi dans le groupe constitué par l'acide oléique, l'acide linoléique, le tributylphosphate et leurs mélanges.

8. Utilisation selon la revendication 7 dans laquelle ledit composé donneur de Lewis est l'acide oléique.

9. Utilisation selon l'une des revendications 6 à 8 dans laquelle ledit solvant comprenant un composé donneur de Lewis comprend également au moins un hydrocarbure contenant au moins 12 atomes de carbone, la proportion (composé donneur de Lewis)/(hydrocarbure + composé donneur de Lewis) étant comprise dans un domaine allant de 1% à moins de 100% poids.

## Patentansprüche

1. Verfahren zur Reinigung eines Einsatzstoffs, der Ethanol, Acetaldehyd und Verunreinigungen umfasst, mit einem Lösungsmittel, das als Lewis-Donor-Lösungsmittel bezeichnet wird:
- einen Schritt A) der Gegenstrom-Flüssig-Flüssig-Extraktion, der am Kopf der Einsatzstoff in Abmischung mit dem aus dem Reextraktionsschritt B) erhaltenen Raffinat, wobei die Mischung die Zuführung des Schritts A) darstellt, und am Sumpf der aus dem Regenerationsschritt C) erhaltene an Lewis-Donor-Lösungsmittel reiche Austragsstrom zugeführt wird, wobei dem an Lewis-Donor-Lösungsmittel reichen Austragsstrom gegebenenfalls ein Zusatz an Lewis-Donor-Lösungsmittel beigemischt wird, und die am Kopf ein Extrakt und am Sumpf einen gereinigten Einsatzstoff produziert und bei einer Temperatur zwischen 10 und 70 °C und einem Druck zwischen 0,1 und 0,5 MPa mit einem Verhältnis von Massendurchflussrate der kontinuierlichen Phase zu Massendurchflussrate der dispersen Phase von weniger als 70 arbeitet;
- einen Schritt B) der Gegenstrom-Flüssig-Flüssig-Reextraktion, der am Kopf ein Hilfslösungsmittel und am Sumpf das aus Schritt A) erhaltene Extrakt zugeführt wird und die am Kopf ein Extrakt und am Sumpf ein Raffinat produziert, wobei das Raffinat dem Schritt A) zugeführt wird, und die bei einer Temperatur zwischen 10 und 70 °C und einem Druck zwischen 0,1 und 0,5 MPa mit einem Verhältnis von Massendurchflussrate der kontinuierlichen Phase zu Massendurchflussrate der dispersen Phase von weniger als 70 arbeitet;
- einen Regenerationsschritt C), in dem das aus Schritt B) erhaltene Extrakt durch eine erste Destillation in ein an leichten Verunreinigungen reiches Destillat und einen Rückstand getrennt wird, wobei der Rückstand einer zweiten Destillation unterworfen wird, welche am Kopf einen an Lewis-Donor-Lösungsmittel reichen Austragsstrom und einen an schweren Verunreinigungen reichen Rückstand produziert;
**dadurch gekennzeichnet, dass** das Lewis-Donor-Lösungsmittel einen Lewis-Donor aus der Gruppe bestehend aus ungesättigten Fettsäuren mit zwischen 12 und 18 Kohlenstoffatomen, Phosphatestern mit zwischen 12 und 30 Kohlenstoffatomen und Mischungen davon umfasst.

2. Verfahren nach dem vorhergehenden Anspruch, wobei die Lewis-Donor-Verbindung aus der Gruppe bestehend aus Ölsäure, Linolsäure, Tributylphosphat und Mischungen davon ausgewählt ist.

3. Verfahren nach Anspruch 1, wobei das Lewis-Donor-Lösungsmittel außerdem mindestens einen Kohlenwasserstoff mit mindestens 12 Kohlenstoffatomen umfasst, wobei das Verhältnis (Lewis-Donor-Lösungsmittel)/(Kohlenwasserstoff + Lewis-Donor-Lösungsmittel) in einem Bereich von 1 bis weniger als 100 Gew.-% liegt.

4. Verfahren nach dem vorhergehenden Anspruch, wobei das Verhältnis in einem Bereich von 15 bis weniger als 100 Gew.-% liegt.

5. Verfahren nach einem der Ansprüche 1 bis 4, wobei es sich bei der Hilfslösungsmittellösung um Wasser handelt.

6. Verwendung eines Lösungsmittels, das eine Lewis-Donor-Verbindung aus der Gruppe bestehend aus ungesättigten Fettsäuren mit zwischen 12 und 18 Kohlenstoffatomen, Phosphatestern mit zwischen 12 und 30 Kohlenstoffatomen und Mischungen davon umfasst, zur Abtrennung von Verunreinigungen aus einem Einsatzstoff, der Ethanol, Acetaldehyd und Verunreinigungen umfasst.

7. Verwendung nach Anspruch 6, wobei die Lewis-Donor-Verbindung aus der Gruppe bestehend aus Ölsäure, Linolsäure, Tributylphosphat und Mischungen davon ausgewählt ist.

8. Verwendung nach Anspruch 7, wobei es sich bei der Lewis-Donor-Verbindung um Ölsäure handelt.

9. Verwendung nach einem der Ansprüche 6 bis 8, wobei das Lösungsmittel, das eine Lewis-Donor-Verbindung umfasst, außerdem mindestens einen Kohlenwasserstoff mit mindestens 12 Kohlenstoffatomen umfasst, wobei das Verhältnis (Lewis-Donor-Lösungsmittel)/(Kohlenwasserstoff + Lewis-Donor-Lösungsmittel) in einem Bereich von 1 bis weniger als 100 Gew.-% liegt.

## Claims

1. Process for the purification of a feedstock comprising ethanol, acetaldehyde and impurities by a solvent, known as Lewis donor solvent:
- a stage A) of counter-currentwise liquid-liquid extraction fed at the top by the said feedstock as a mixture with the raffinate resulting from the re-extraction stage B), this mixture constituting the feed of the said stage A), and at the bottom by the effluent rich in Lewis donor solvent resulting from the regeneration stage C), a contribution of Lewis donor solvent optionally being mixed with the said effluent rich in Lewis donor solvent, and producing an extract at the top and a purified feedstock at the bottom, carried out at a temperature of between 10 and 70°C and at a pressure of between 0.1 and 0.5 MPa, with a flow rate by weight of continuous phase/flow rate by weight of disperse phase ratio of less than 70;
- a stage B) of counter-current liquid-liquid re-extraction fed at the top by an auxiliary solvent and at the bottom by the extract resulting from stage A), and producing an extract at the top and a raffinate at the bottom, the said raffinate feeding the said stage A), carried out at a temperature of between 10 and 70°C and at a pressure of between 0.1 and 0.5 MPa, with a flow rate by weight of continuous phase/flow rate by weight of disperse phase ratio of less than 70;
- a stage C) of regeneration in which the extract resulting from stage B) is separated by a first distillation into a distillate rich in light impurities and a residue which undergoes a second distillation, which produces an effluent rich in Lewis donor solvent at the top and a residue rich in heavy impurities; **characterized in that**
the said Lewis donor solvent comprises a Lewis donor chosen from the group consisting of unsaturated fatty acids having between 12 and 18 carbon atoms, phosphate esters having between 12 and 30 carbon atoms and their mixtures.

2. Process according to the preceding claim, in which the said Lewis donor compound is chosen from the group consisting of oleic acid, linoleic acid, tributyl phosphate and their mixtures.

3. Process according to Claim 1, in which the said Lewis donor solvent also comprises at least one hydrocarbon containing at least 12 carbon atoms, the (Lewis donor compound)/(hydrocarbon + Lewis donor compound) proportion being within a range from 1% to less than 100% by weight.

4. Process according to the preceding claim, in which the said ratio is within a range from 15% to less than 100% by weight.

5. Process according to one of Claims 1 to 4, in which the said solution of auxiliary solvent is water.

6. Use of a solvent comprising a Lewis donor compound chosen from the group consisting of unsaturated fatty acids having between 12 and 18 carbon atoms, phosphate esters having between 12 and 30 carbon atoms and their mixtures in the separation of the impurities from a feedstock comprising ethanol, acetaldehyde and impurities.

7. Use according to Claim 6, in which the said Lewis donor compound is chosen from the group consisting of oleic acid, linoleic acid, tributyl phosphate and their mixtures.

8. Use according to Claim 7, in which the said Lewis donor compound is oleic acid.

9. Use according to one of Claims 6 to 8, in which the said solvent comprising a Lewis donor compound also comprises at least one hydrocarbon containing at least 12 carbon atoms, the (Lewis donor compound)/(hydrocarbon + Lewis donor compound) proportion being within a range from 1% to less than 100% by weight.
